Europäisches Patentamt

European Patent Office

Office européen des brevets

(11) Veröffentlichungsnummer : **0 080 166**
**B1**

(12) **EUROPÄISCHE PATENTSCHRIFT**

(45) Veröffentlichungstag der Patentschrift :
28.08.85

(51) Int. Cl.⁴ : **C 12 P 17/18**

(21) Anmeldenummer : **82110641.6**

(22) Anmeldetag : **18.11.82**

(54) **Isolierung von chemisch instabilen Antibiotika aus Fermentationslösungen.**

(30) Priorität : **21.11.81 DE 3146190**

(43) Veröffentlichungstag der Anmeldung :
**01.06.83 Patentblatt 83/22**

(45) Bekanntmachung des Hinweises auf die Patenterteilung : **28.08.85 Patentblatt 85/35**

(84) Benannte Vertragsstaaten :
**AT BE CH DE FR GB IT LI NL**

(56) Entgegenhaltungen :
**EP-A- 0 024 447**
**DE-A- 2 528 622**
**DE-A- 2 652 678**
**DE-A- 2 808 636**
**US-A- 4 000 161**

(73) Patentinhaber : **HOECHST AKTIENGESELLSCHAFT**
**Postfach 80 03 20**
**D-6230 Frankfurt am Main 80 (DE)**

(72) Erfinder : **Huber, Gerhard, Dr.**
**In den Bleichwiesen 2**
**D-6233 Kelkheim (Taunus) (DE)**
Erfinder : **Schindler, Peter, Dr.**
**Reinhardswaldweg 1**
**D-6082 Mörfelden-Walldorf (DE)**

## Beschreibung

Von Streptomyces-Stämmen gebildete β-Lactam-Antibiotika mit Carbapenem-Strukturen, z. B. die Thienamycin- und Olivansäuregruppe, zeichnen sich durch besonders hohe antimikrobielle Aktivität und ein breites Wirkungsspektrum aus. Gleichzeitig sind die Antibiotika dieser Gruppe hoch wirksame Inhibitoren bzw. Inaktivatoren von β-Lactamasen verschiedener Herkunft. Die Isolierung dieser Substanzen aus den Fermentationslösungen ist infolge ihrer extremen chemischen Instabilität außerordentlich erschwert. So liegen die Halbwertzeiten des chemischen Abbaus in wäßrigen Lösungen im mittleren pH-Bereich (pH 6-8) für Thienamycin je nach Bedingungen zwischen 0,3 und 6 Stunden (J. Antibiot. 32, 1-12, 1979), bei Olivansäuren zwischen 4 und 27 Stunden (J. Antibiot. 32, 295-304, 1979). Die Instabilität wird bei höheren Antibiotika-Konzentrationen, wie sie im Verlauf der Isolierung und Anreicherung auftreten, noch weiter verstärkt. Die Folge ist der Verlust eines großen Anteils der antibiotischen Aktivität im Verlauf des Isolierungsverfahrens. Diese Verluste werden noch erhöht durch die Tatsache, daß die Aktivität in der Fermentationslösung nach Erreichen des Maximums und Beendigung der Produktbildung sehr schnell abfällt, was vor allem auf die bei der erhöhten Fermentationstemperatur (28-30 °C) verstärkte Instabilität der Carbapenem-Antibiotika zurückzuführen ist. Die zu Beginn der Isolierung normalerweise durchgeführte Filtration des Streptomyces-Mycels führt vor allem bei größeren Fermentationsansätzen, die nicht schnell genug gekühlt werden können, zu erheblichen Aktivitätsverlusten. So werden nach der in dieser Gruppe üblichen ersten Anreicherungsstufe mittels Ionenaustauscher (siehe zitierte Literatur) Aktivitätsausbeuten von 10-40 % erhalten.

Es erschien daher als wünschenswert, Fermentationen der besagten Antibiotika am Maximum der Produktbildung zu stoppen, und die gesamte Aktivität unmittelbar in eine stabiliserte Form überzuführen.

Es wurde nun überraschenderweise gefunden, daß die Unterbrechung der Fermentationen am gewünschten Zeitpunkt und Überführung in eine stabilisierte Form dadurch erzielt werden kann, daß man die Antibiotika beim Maximum der Produktbildung an geeignete Anionenaustauscherharze adsorbiert und das Harz von der Fermentationslösung abtrennt.

Die Adsorption kann z. B. so erfolgen, daß man zu der Fermentationslösung ein die antibiotische Aktivität bindendes Anionenaustauscherharz zufügt und die Fermentationslösung beispielsweise durch Verdünnen mit kaltem Wasser schnell auf etwa 1-10, vorzugsweise 1-6 °C abkühlt.

Noch vorteilhafter ist es, das Anionenaustauscherharz in kaltem Wasser von 1-10, vorzugsweise 1-6 °C zu suspendieren und die Fermentationslösung in diese Suspension einzupumpen, wobei eine schnelle Abkühlung und sofortige Bindung der antibiotischen Aktivität erzielt wird. Als Nebeneffekt wird die Fermentationslösung so gleichzeitig mit Wasser verdünnt.

Als Anionenaustauscherharze eignen sich im vorliegenden Falle vorzugsweise solche mit mittlerer bis starker Basizität und mit geringer Vernetzung und poröser Struktur wie z. B. Dowes® 1 × 2, Amberlite® IRA-401 S, Amberlite® IRA-900.

Die Abtrennung des Austauschers von Fermentationslösung und Mycel kann durch Sedimentation (Absetzen) aus der wie oben verdünnten Fermentationslösung erfolgen. In einer anderen Ausführungsform wird die Ionenaustauscher und Mycel enthaltende verdünnte Fermentationslösung über ein geeignetes Sieb, z. B. ein Vibrationssieb (z. B. ®Perflux), das den Ionenaustauscher zurückhält und das Mycel passieren läßt, filtriert.

Zur Erzielung einer möglichst geringen Sedimentationszeit soll der Ionenaustauscher so gewählt werden, daß die Dichte des Harzes wesentlich größer ist als die des Mycels, außerdem ist ein möglichst großer Korndurchmesser erforderlich z. B. 20-50 mesh, entsprechend 300-800 μm, bei handelsüblichen Harzen.

Bei Anwendung eines geeigneten Harzes und Vorgehen nach der oben beschriebenen Verfahrensweise werden mehr als 90 % der in der Fermentationslösung gebildeten Aktivität an den Ionenaustauscher gebunden. Das Harz kann sofort in der üblichen Weise weiterverarbeitet werden oder es kann bis zu einer weiteren Verarbeitung nach Absaugen der überschüssigen Feuchtigkeit eingefroren und bei etwa − 20 °C aufbewahrt werden. In diesem Zustand sind die gebundenen Antibiotika mindestens 2-3 Monate stabil.

Zur Isolierung der antibiotischen Aktivität wird das Ionenaustauscherharz in bekannter Weise mit Salzlösungen, z. B. Natriumchlorid, Natriumacetat oder Kaliumchlorid eluiert, wobei zur Erhöhung der Elutionsausbeute, die etwa 55-80 % betragen kann, ggf. ein mit Wasser mischbares organisches Lösungsmittel. wie z. B. Methanol, Isopropanol oder Aceton, vorzugsweise mit einer Konzentration von 20-80 %, zugesetzt wird.

Die Gesamtausbeute des ersten Reinigungsschritts beträgt somit etwa 50-75 %, bezogen auf die Aktivität in der Fermentationslösung.

Die Aktivität der Thienamycin- und Olivansäure-Antibiotika kann in üblicher Weise durch mikrobiologische Tests (Agardiffusionstest, turbidimetr. Test) mit geeigneten Testkeimen, z. B. Escherichia coli, Bacillus subtilis, bestimmt werden. Als zweckmäßiger Schnelltest eignet sich besonders die Bestimmung der β-Lactamase-Inhibitor-Wirkung, z. B. mit Hilfe der neuen chromogenen β-Lactamase-Substrate PADAC und CENTA (P. Schindler et al, Abstracts 21. Intersience Conference on Antimicrobial Agents and Chemotherapy, Chicago, 1981). Die nach diesen Methoden bestimmte Aktivität wird in Enzym-Inhibitor-Einheiten (UE 50/ml) angegeben.

Beispiel 1

10 l Fermentationslösung des Stammes Streptomyces Y 5633A (DSM 2323), der mehrere Olivansäure-Komponenten bildet und bei der Fermentation (siehe z. B. J. Antibiot. *32*, 295-304, 1979) nach 42 Stunden das Maximum der Aktivität, gemessen in Enzym-Inhibitor-Einheiten, mit 15 UE 50/ml = 150 000 UE 50 erreicht, werden durch Zugabe von kaltem Wasser unter Rühren und gleichzeitiger Abkühlung auf 6 °C zu diesem Zeitpunkt mit 300 g Anionenaustauscherharz Dowex® 1 × 4, 20-50 mesh, versetzt. Nach 30 Min. wird die Fermentationslösung in einen trichterförmigen Behälter mit Bodenablaß gefüllt und das Austauscherharz absitzen gelassen. Das nach ausreichender Sedimentation über den Boden abgezogene Harz wird dekantierend zur Entfernung der Mycelreste mit Wasser gewaschen und dann in üblicher Weise, z. B. durch Elution, weiterverarbeitet. Das Harz enthält 138 000 UE 50 (O 92 %), in der abgetrennten Fermentationslösung werden 12 000 UE 50 (= 8 %) gemessen. Das gewonnene Adsorbat wird unter Kühlung und Rühren mit 1,2 l einer Lösung von 6 % KCl in 50 %igem Aceton behandelt und das Harz nach 3 Stunden durch Filtration abgetrennt. Die Elutionslösung enthält 93 000 UE 50 (= 62 %) und kann nach den üblichen Methoden weiterverarbeitet werden.

Beispiel 2

175 l Fermentationslösung des Streptomyceten-Stammes Y 5633A (DSM 2323) wird sofort nach Erreichen der maximalen antibiotischen Aktivität von 22,0 UE 50/ml, entsprechend 3 850 000 UE 50 in eine Vorlage mit Bodenventil gepumpt, die 6 l Anionenaustauscher Amberlite® IRA-401 S, 20-50 mesh, in 400 l Wasser von 6 °C suspendiert, enthält. Nach einstündigem Rühren läßt man das Harz etwa 30 Minuten sedimentieren und zieht es über das Vodenventil der Vorlage über ein Sieb mit der lichten Maschenweite von 150 μm ab. Das auf dem Sieb zurückgehaltene Austauscherharz wird in einem Behälter bis zur Entfernung der Mycelanteile dekantierend mit kaltem Wasser (6 °C) gewaschen und oberflächlich trocken gesaugt. Das Harz (3,6 kg) enthält 3 620 000 UE 50 (= 94 %), in der Fermentationslösung sind noch 230 000 UE 50 (= 6 %) enthalten.

Das so gewonnene Adsorbat kann bei − 20 °C mindestens 2 Monate ohne Aktivitätsverlust gelagert werden.

Zur Elution wird das Ionenaustauscherharz in eine Säule mit Kühlmantel gefüllt und unter Kühlung (6 °C) mit 6 % Kaliumchlorid in 50 %igem Aceton eluiert. Die Fraktionen zu 500 ml werden im Inhibitortest auf Aktivität geprüft. Die Hauptaktivität (2 462 000 UE 50 = 68 %) befindet sich in den Fraktionen 12-20. Die Fraktionen werden vereinigt, im Hochvakuum auf 0,9 l eingeengt, und auf eine gekühlte Säule von 6 l Adsorptionsharz Diaison HP-20 gegeben und die Aktivität mit kaltem Wasser eluiert. Die aktiven salzfreien Fraktionen (10-14), die 2 136 000 UE 50 = 59 % enthalten, werden vereinigt und lyophilisiert. Das hierbei gewonnene rohe Antibiotikagemisch wird in bekannter Weise weiter gereinigt.

Beispiel 3

Der Olivansäuren produzierende Streptomyceten-Stamm Y 5633 A (DSM 2323) wird in 3 l Nährlösung in üblicher Weise gezüchtet und die Fermentationslösung nach 38 Stunden bei Erreichen des Maximums der Produktbildung zu einer auf 4 °C gekühlten Suspension von 90 g Amberlite® IRA-401 S, 20-50 mesh, in 6 l Wasser gegeben und das Harz in der im Beispiel 1 beschriebenen Weise abgetrennt. Das Harz enthält 90 % der in der Fermentationslösung gemessenen Aktivität.

Zur Elution der Aktivität wird der Amberlite IRA-401 S in eine Säule (2,5 × 30 cm) gefüllt und die Aktivität mit einem linearen Gradienten von 50 % Aceton und 8 % KCl in 50 % Aceton eluiert (Fraktionengröße 20 ml). Die Hauptaktivität wird in den Fraktionen 11-12 gefunden, die Elutionsausbeute beträgt 55 %.

**Patentansprüche**

1. Verfahren zur Isolierung von chemisch instabilen Carbapenem-Antibiotika aus Fermentationslösungen, dadurch gekennzeichnet, daß die gebildeten Antibiotika beim Maximum der Produktbildung an geeignete Anionenaustauscherharze adsorbiert, hierdurch in eine stabilisierte Form überführt und das Harz von der Fermentationslösung abgetrennt wird.

2. Verfahren nach Anspruch 1, dadurch gekennzeichnet, daß man als Anionenaustauscherharze solche mit mittlerer bis starker Basizität verwendet.

3. Verfahren nach Anspruch 2, dadurch gekennzeichnet, daß die Anionenaustauscherharze eine Korngröße von vorzugsweise 20-50 mesh besitzen.

4. Verfahren nach Anspruch 1, dadurch gekennzeichnet, daß man das Anionenaustauscherharz am geeigneten Zeitpunkt der Fermentationslösung zufügt und diese schnell abkühlt.

5. Verfahren nach Anspruch 1, dadurch gekennzeichnet, daß man die Fermentationslösung in eine Vorlage pumpt, in der sich das Anionenaustauscherharz, in kaltem Wasser suspendiert, befindet.

6. Verfahren nach Anspruch 1, dadurch gekennzeichnet, daß man die Anionenaustauscher von der Fermentationslösung durch Sedimentation abtrennt.

7. Verfahren nach Anspruch 1, dadurch gekennzeichnet, daß man die Anionenaustauscher von der Fermentationslösung durch Filtration über ein geeignetes Sieb, das den Austauscher zurückhält und das Mycel passieren läßt, abtrennt.

## Claims

1. A process for isolating chemically unstable carbapenem antibiotics from fermentation solutions, which comprises adsorbing the antibiotics formed at the maximum of the product formation on suitable anion exchanger resins, thus converting them to a stabilized form and separating the resin from the fermentation solution.

2. The process of claim 1, which comprises using as anion exchanger resins such of medium to strong basicity.

3. The process of claim 2, wherein the anion exchanger resins have a particle size of preferably 20-50 mesh.

4. The process of claim 1, which comprises adding the anion exchanger resin to the fermentation solution at an adequate moment and cooling said solution rapidly.

5. The process of claim 1, which comprises pumping the fermentation solution into a recipient containing the anion exchanger resin suspended in cold water.

6. The process of claim 1, which comprises separating the anion exchangers from the fermentation solution by sedimentation.

7. The process of claim 1, which comprises separating the anion exchangers from the fermentation solution by filtration on an appropriate sieve retaining the exchanger and allowing the passage of the mycelium.

## Revendications

1. Procédé d'isolement d'antibiotiques du Carbapénème chimiquement instables de solutions de fermentation, caractérisé en ce que les antibiotiques formés sont adsorbés au maximum de la formation de produit sur des résines échangeuses d'anions appropriées, amenés par ce moyen sous une forme stabilisée et en ce que la résine est séparée de la solution de fermentation.

2. Procédé suivant la revendication 1, caractérisé en ce qu'on utilise comme résines échangeuses d'anions des résines ayant une basicité moyenne à forte.

3. Procédé suivant la revendication 2, caractérisé en ce que les résines échangeuses d'anions possèdent de préférence une granulométrie de 300 à 800 μm.

4. Procédé suivant la revendication 1, caractérisé en ce qu'on ajoute la résine échangeuse d'anions au moment approprié à la solution de fermentation, et en ce qu'on refroidit rapidement celle-ci.

5. Procédé suivant la revendication 1, caractérisé en ce qu'on pompe la solution de fermentation dans un récipient dans lequel se trouve la résine échangeuse d'anions, en suspension dans de l'eau froide.

6. Procédé suivant la revendication 1, caractérisé en ce qu'on sépare les échangeurs d'anions de la solution de fermentation par sédimentation.

7. Procédé suivant la revendication 1, caractérisé en ce qu'on sépare les échangeurs d'anions de la solution de fermentation par filtration sur un tamis approprié, qui retient l'échangeur et laisse passer le mycélium.